Europäisches Patentamt

(19) European Patent Office

Office européen des brevets

(11) Publication number: **0 372 969**
**A1**

## (12) EUROPEAN PATENT APPLICATION

(21) Application number: **89312780.3**

(22) Date of filing: **07.12.89**

(51) Int. Cl.⁵: **A61L 31/00**

(30) Priority: **07.12.88 US 280928**

(43) Date of publication of application:
**13.06.90 Bulletin 90/24**

(84) Designated Contracting States:
**AT CH DE ES FR GB IT LI SE**

(71) Applicant: **JOHNSON & JOHNSON PATIENT CARE, INC.**
**501 George Street**
**New Brunswick New Jersey 08903(US)**

(72) Inventor: **Linsky, Cary**
**25 Beacon Hill Drive**
**E. Brunswick, NJ 08816(US)**
Inventor: **Cunningham, Timothy**
**Box 374 Cherryvill Hollow Road**
**Flemington, NJ 08822(US)**
Inventor: **Pines, Eli**
**100 Deer Run**
**Watchung, NJ 07060(US)**

(74) Representative: **Fisher, Adrian John et al**
**CARPMAELS & RANSFORD 43 Bloomsbury Square**
**London WC1A 2RA(GB)**

(54) Low molecular weight heparin, heparinoid and hexuronyl hexosaminoglycan sulfate containing adhesion prevention barrier and process.

㊼ An improved adhesion-preventative barrier fabric comprising an oxidized regenerated cellulose (ORC) fabric (or matrix having equivalent properties) which is drapable, conformable, adherent to body organs, and substantially absorbable within thirty (30) days in the body, which fabric has low molecular weight heparin, heparinoid or hexuronyl hexosaminoglycan sulfate active absorbed thereon, said active being present in a non-toxic, adhesion-preventative, effective amount and potency; and, the process of using said improved barrier fabric or matrix to administer low molecular weight heparin, heparinoid or hexuronyl hexosaminoglycan sulfate to prevent surgical adhesions.

EP 0 372 969 A1

# LOW MOLECULAR WEIGHT HEPARIN, HEPARINOID AND HEXURONYL HEXOSAMINOGLYCAN SULFATE CONTAINING ADHESION PREVENTION BARRIER AND PROCESS

## Field of the Invention

This invention relates to adhesion barrier materials useful in surgery for preventing post operative adhesions; and is more particularly concerned with a matrix which is drapable, conformable, adherent to body organs, substantially absorbable within thirty (30) days in the body, said matrix having an adhesion-preventative amount of hexuronyl hexosaminoglycan sulfate, heparinoid or low molecular weight heparin incorporated therein, and is still more particularly concerned, as a preferred version, with an absorbable matrix, such as a fabric of oxidized regenerated cellulose (ORC) which is impregnated with an adhesion-preventative amount and potency of hexuronyl hexosaminoglycan sulfate, heparinoid or low molecular weight heparin, and to the process of administering heparin topically to an internal body organ by absorbing it on an ORC fabric which is applied to the body organ during surgery.

## Background of Invention

Post operative adhesions represent a major problem in patients recovering from surgery. When organs and tissues are subject to surgical and related trauma, there is a tendency for adhesions to form between the affected areas and neighboring tissue.

In the case of intestinal surgery, the incidence of adhesions causing intestinal obstructions has been reported as approximately four times that due to strangulated hernia. The post operative formation or reformation of pelvic adhesions is reported to be a major factor contributing to the relatively poor results obtained in infertility surgery.

Various methods have been suggested for reducing the incidence of peritoneal adhesions following surgical intervention, but results have not been entirely favorable. One method involves the application of chemical treating agents to the site of the surgical incision or abrasion in an effort to inhibit the physiological response responsible for the formation of the fibrous tissue which comprises the adhesion mass. In this category are enzymes such as fibrinolysin and papase, polyphloretinphosphate, oxyphenbutazone, a mixture of phenylbutazone and prednisolone, polyvinylpyrrolidone and dextran.

A second approach to preventing the formation of adhesions is to install a physical barrier material between the site of the surgical activity and the neighboring tissue where adhesions are most expected to occur. In this category are silicone elastomer sheets, absorbable gelatin film, and knit fabrics of oxidized regenerated cellulose (ORC). Attempted use of these prior art materials and methods are reported by Larssen, Acta Chir Scand 144: pp. 375-378 (1978) and Raftery, Br. J. Surg. Vol. 67 pp. 57-58 (1980); Schroder, Acta Chir Scand 148 pp. 595-596 (1982), Yemini, Int. J. Fertil 29 pp. 194-196 (1984) and Soules, Am. J. Obstet & Gyn. Vol. 143 pp. 829-834 (1982); and Nishimura, Jpn. J. Surg. Vol. 13 pp. 159-163 (1983).

In the copending U.S. patent application of Linsky and Cunningham, Serial No. 768,280, * filed August 22, 1985, entitled "Method and Material for Prevention of Surgical Adhesions," the teachings of which are specifically incorporated herein by reference, a particular fabric construction was disclosed resulting in an improved adhesion barrier fabric. That material was a fabric of oxidized regenerated cellulose (ORC) characterized by having a porosity as defined by an open area of 12 to 20 percent and a density of from about 8 to 15 mg/cm². A typical fabric is prepared from 60 denier, 18 filament bright rayon yarn knitted on a 32 gauge 2 bar warp knitting machine. The knit fabric is oxidized using conventional procedures as described for example in U.S. Patent No. 3,364,200.

The above fabric was effective in reducing the incidence of postoperative adhesions when positioned as a physical barrier between the site of the surgical activity and neighboring tissue, but further improvement is still possible. Test results using a preferred version of the above fabric, called TC-7, in adhesion reduction in a rabbit uterine horn model, established its ease of handling. It proved simple to apply, conformed well to the structure, adhered in place, and substantially absorbed within two weeks after surgery.

Heparin including low molecular weight heparin is normally administered intravenously or subcutaneously, not topically. In an article entitled "Heparin Releasing Antiadhesive Membranes" by Y. Noishiki and T. Miyata published in Jinko Zoki, 14(2), pp. 788-791 (1985), a collagen membrane (special treated

*See EP-A-0213563

human amnion) having protamine cross-linked into the collagen network was immersed in 1% heparin solution so the heparin was ionically bound to the protamine which had been cross-linked in the collagen. The resultant heparinized collagen membrane was stitched into place covering a wound on the serosal membrane of the large intestines of dogs. The animals were examined after 3 days, 60 days, 173 days and 687 days. No signs of adhesions were found. The collagen membrane was not biodegradable, since much of it remained even after 687 days. The heparin was released slowly and steadily, so that 76% of the heparin originally present in the membrane was released over a period of three months.

Applicants' invention differs from the above article: in using a biodegradable matrix or fabric as a carrier for heparin, low molecular weight heparin, heparinoid or hexaronyl hexosaminoglycan sulfate; the ORC fabric or other matrix is macroscopically broken down within thirty (30) days or less; in not requiring affixing the carrier matrix or ORC fabric with sutures, but rather in using a pliable, conformable matrix or fabric which remains in place without need for sutures; in not exerting a systemic effect but only a local effect provided by the active soaked matrix or fabric of the present invention wherein all the active is released during the first week rather than slowly and steadily over many months; and in the fact that the heparin, low molecular weight heparin, heparinoid and hexuronyl hexosaminoglycan sulfate is not ionically bound to the carrier, but is only absorbed on the matrix or fabric.

## Summary of the Invention

We have now found unexpectedly that even greater improved results in reducing postoperative adhesions are obtained when a drapable, conformable adhesion barrier fabric constructed of a bioresorbable material, such as the ORC knitted fabric disclosed in the aforesaid copending U.S. Patent Application Serial No. 768,280 (hereinafter called TC-7), or other matrix having similar properties, is impregnated with heparin, as well as, low molecular weight heparin, heparinoid and hexuronyl hexosaminoglycan sulfate (HHS). We have further found that such improved results occur when low molecular weight (LMW heparin), heparinoid and HHS is used to impregnate even the less effective ORC barrier fabrics, such as Surgicel*. This is especially surprising since heparin, low molecular weight heparin, heparinoid and HHS alone, e.g., in lavage solution, is not effective to prevent adhesions. LMW heparin, heparinoid and HHS act as adhesion-preventing medicaments or actives when incorporated into the matrix of the present invention. A preferred embodiment of the matrix is ORC fabric. The LMW heparin, heparinoid and HHS may be added to the matrix, e.g. ORC fabric, either before or during the surgery.

One advantage of adding the heparin to the barrier fabric is that the fabric absorbs and holds small quantities of LMW heparin, heparinoid and HHS, so no excess amount of active is used which the body would have to absorb or eliminate. This allows much smaller amounts to be used, and is important when dealing with a potentially toxic substance. It ensures minimal LMW heparin, heparinoid or HHS is spilled into or onto other organs or body cavities. By using the matrix or barrier fabric to deliver the actives to the specific place in the body where it is needed and especially intraperitoneally, the following important benefits are realized: localized delivery, smaller doses, maximum efficacy, minimum side effect, and reduction of lag time to build drug concentration.

The present invention includes both product and related process aspects. In its product aspect, it involves: a matrix (as defined below) having an adhesion-preventative amount and potency of LMW heparin, heparinoid or HHS incorporated therein; and also, an improved adhesion-preventative barrier fabric comprising an oxidized regenerated cellulose fabric which is drapable, conformable, adherent to body organs, and substantially absorbable within thirty (30) days in the body, which fabric has heparin absorbed thereon, said LMW heparin, heparinoid or HHS being present in a non-toxic, adhesion-preventative, effective amount and potency. Other matrices which could be used should have properties equivalent to the oxidized regenerated cellulose fabric. Among these are included, for illustrative and not exclusionary purposes, materials such as hyaluronic acid, cross-linked and uncross-linked collagen webs, synthetic resorbable polymers, gelatin films, absorbable gel films, oxidized cellulose fabrics, films and the like, when fabricated into a form which is drapable, conformable, adherent to body organs, substantially absorbable within thirty (30) days in the body, capable of absorbing LMW heparin, heparinoid or HHS, and safe for use is surgery.

In its process aspects, the present invention involves the process of preventing surgical adhesions which comprises positioning as a physical barrier, between the site of the surgical activity and neighboring tissue, a LMW heparin, heparinoid or HHS containing matrix, (as defined above) preferably in the form of an oxidized regenerated cellulose adhesion-preventative barrier fabric. Further a process is provided of administering LMW heparin, heparinoid or HHS topically to an internal body organ during surgery for the purpose of preventing surgical adhesions which comprises: applying an oxidized regenerated cellulose

3

fabric (or other matrix as defined above) containing LMW heparin, heparinoid or HHS absorbed on it to the outer surface of an internal body organ, said fabric (or other matrix) being drapable, conformable, adherent to body organs, and substantially absorbable within thirty (30) days in the body. It also involves the process of delivering LMW heparin, heparinoid or HHS topically to a particular organ in the body by absorbing that drug on an ORC barrier fabric or similar type of absorbable matrix, which is then applied to the outer surface of said organ.

Detailed Description of the Invention

The matrix useful in the present invention may be an ORC fabric or be made of any non-ORC material having the characteristics described in connection with the ORC fabric below. Various useful matrices have been mentioned above, and others will be apparent to persons skilled in the art. The preferred matrix useful in the present invention is an oxidized regenerated cellulose (ORC) fabric which is drapable, conformable, adherent to body organs, and substantially absorbable within thirty (30) days in the body. The term "substantially absorbable within 30 days", means that macroscopically by gross observation there is no residual material remaining when the area of the body where the barrier fabric was placed is inspected at the time specified. Such a fabric is easy to apply and will stay in place on the organ without the use of sutures. It is biocompatible and resorbable.

The ORC fabric preferably is knit but could be fabricated in other forms, e.g., nonwoven, or woven, if desired. Commercially available forms of ORC fabric include the following: SURGICEL and SURGICEL NU-KNIT brand of absorbable hemostat which are described in the PDR (Physicians' Desk Reference), 1986 edition. The TC-7 fabric previously identified above is also knitted. It is currently being clinically tested.

For convenience, the description of the present invention will mainly refer to the most preferred embodiment, it being understood that the other matrix materials, which are regarded as less preferred embodiments, would be used in a similar manner.

Heparin (which term is intended to include salt forms, such as the calcium or sodium salt) is a mixture of naturally occurring mucopolysaccharides of varying molecular weight found in various organs. Heparin is isolated by numerous extraction methods to form molecular, chemically, and/or enzymatically depolymerized mixtures.

Heparin represents a heterogenous mixture of polysaccharides and oligosaccharides with a molecular weight range of 1200 to 40,000 daltons with a mean molecular weight of approximately 15,000 daltons. Depending upon the source and method of manufacturing, 10 to 30 molecular species may be present in a given heparin preparation. Fractionation of heparin has been proposed as a means of obtaining lower molecular weight heparin fractions with defined chemical and biologic characteristics. The molecular weight of these fractions ranges from 2500 to 10,000 daltons. A typical or average product would have a molecular weight of about 5000 daltons.

The development of low molecular weight fractions of heparin are defined in terms of molecular weight and biologic activity. These fractions provide an alternate to heparin in conditions in which heparin may exert toxic effects, e.g. hemorrhage. Such lower molecular weight fractions of heparin are identified herein as low molecular weight heparin (LMW heparin) available in different potencies, referred to as USP Heparin Units. LMW heparin potencies useful in the present invention may range from 100 to 10,000 USP Heparin Units. Potencies of 100 to 2,000 units per six (6) square inches of fabric are particularly useful, with 500 to 1,500 being preferred.

LMW heparin, heparinoid and HHS is preferably used in the same liquid form as would be used for administration by injection, e.g., as LMW Heparin Sodium Injection. Use in this form easily provides the desired number of USP Units in sufficient liquid to be absorbed on the fabric. Typically 1 ml of LMW Heparin Sodium, heparinoid or HHS Injection will wet and saturate or almost saturate a 2"x3" piece of ORC fabric without running off or being visible in the knitted fabric interstices.

Heparinoid is a by-product of heparin manufacture and consists of glycosamino-glycans and displays antithrombotic/anticoagulant activity. Heparinoid is more fully described in Ten Cate, J. W. et al. "Low Molecular Weight Heparins and Heparinoid, A Recent Development in Anticoagulent Treatment: Application in Hemodialysis", The International Journal of Artificial Organs, Vol. 9, No. 6, Pg. 397-400 (1985), the entire disclosure of which article is hereby incorporated herein by reference.

Hexuronyl Hexosaminoglycan Sulfate (HHS) is described in Brit. Pat. 33615 (1977) and ext., U.S. Pat. 4,320,699 and U.S. Pat. 4,264,733. HHS is a biological fraction of hexuronyl hemosaminoglycan sulfates (mostly heparan sulphate and dermatan sulphate) extracted and purified from animal organs. It is a white amorphous and hygroscopic powder which is freely soluble in water, diluted mineral acids and alkalis, but insoluble in ethanol. HHS is stated by the above-identified references to show good anti-thrombotic activity, comparable to heparin; low total anti-clotting activity (about 1/3 of heparin); can act as a "clearing" factor by lowering the blood levels of triglycerides and cholesterol; has a low acute toxicity (LD50); and does not affect platelet aggregation.

The LMW heparin, heparinoid and HHS which is absorbed on the ORC fabric (or other matrix material) may be applied to the fabric in liquid form at the time of actual use on a body organ during surgery, i.e., the fabric is cut to the desired size and draped on and conformed to the body organ, and LMW heparin, heparinoid or HHS is then applied, in a non-toxic, adhesion-preventative amount and potency, via syringe or pipette or the like to said fabric. The amount of LMW heparin, heparinoid or HHS utilized normally should be sufficient to saturate the fabric but not so great that any active drips off or is spilled into or onto other organs or body cavities.

The LMW heparin, heparinoid or HHS. active alternatively may be absorbed on the ORC fabric (or other matrix materials) and then dried. The dried active impregnated fabric may then be applied to the body organ during surgery. Moisture from the body organ, or a solution (e.g. Ringer's, saline or water) can be applied to quickly rehydrate and return the active to a liquid form. Using this alternative, the ORC fabric with LMW heparin, heparinoid or HHS absorbed on it, would be dried in any desired manner, e.g., air-dried, freeze-dried, oven-dried. vacuum-dried etc., after which it would be sealed in any desired type of sealable pouch or container customarily used to contain sterile surgical products such as dressings, sutures, etc., and then sterilized. Mylar foil laminates may be used as the pouch material and radiation sterilization may be used to carry out this alternative. The LMW heparin, heparinoid or HHS impregnated barrier fabric (or matrix) can thus be made easily available for use in surgery in a variety of sizes. Combinations or mixtures of any or all of heparin, LMW heparin, heparinoid, and/or HHS may be applied to the adhesion preventing barrier fabric or matrix.

The efficacy of various LMW heparin, heparinoid, and HHS impregnated barrier fabrics of the present invention, as compared to otherwise identical non-heparin impregnated fabrics, was determined by the uterine horn scrape procedure as described in the disclosure of co-pending application which is hereby incorporated herein by reference, U.S.S.N. 912,450, which the scoring system consists of the following grading:

0 = No adhesions
1 = 25% of traumatized area
2 = 50% of traumatized area
3 = Total involvement

Fractional scores are given for extent of adhesions intermediate between the above grades. The severity (tenacity) of the adhesions are measured as follows:

0 = No resistance to separation
0.5 = Some resistance (moderate force required)
1 = Sharp dissection needed

The total grade thus is additive giving a range of adhesion scores of 0 - 4 which represents both extent and severity.

In the following examples, all the adhesion results listed were obtained by the above procedure, or by the above procedure without any heparin, LMW heparin, heparinoid or HHS.

Example 1: TC-7, With LMW Heparin Added In Situ

TC-7 fabric in 2x3 inch pieces is impregnated with LMW Heparin Sodium (Hepar - RD Heparin - 5mg/ml 490 USP Units/ml). One ml of solution is used to saturate each piece after the fabric is applied to a uterine horn.

This effect of combining LMW heparin with this fabric is evaluated by the uterine horn procedure and the results are:

| Untreated Controls | Heparin Impregnated Fabric |
|---|---|
| 4 | 0 |
| 4 | 0 |
| 0 | 0 |
| 2 | 0 |
|  | 0 |
|  | 0 |
|  | 0 |
|  | 0 |
|  | 0 |
|  | 0 |
|  | 0 |
|  | 0 |
| $\overline{X} = 2.5$ | $\overline{X} = 0.00$ |
| 2 animals | 6 animals |
| 4 horns | 12 horns |

As can be seen, the untreated controls have an average adhesion of 2.5 while all of the animals with the above LMW heparin-impregnated TC-7 fabric had no adhesions.

Examples 2 and 3

In Examples 2 and 3, the procedure of Example 1 is changed so that HHS is substituted for LMW heparin and applied to TC-7 fabric. The concentration of HHS added is 4 mg/ml for Example 2 and 20 mg/ml for Example 3. The impregnated fabrics were then tested by the uterine horn procedure and the results were:

| Untreated Control | TC-7 + 4mg/ml HHS | TC-7 + 20mg/ml HHS |
|---|---|---|
| 4 | 1 | 0 |
| 1.5 | 0 | 0 |
| 0 | 0 | 0 |
| 0 | 0 | 0 |
| 4 | 0 | 0 |
| 4 | 0 | 0 |
| 0 | 0 | 0 |
| 2 | 0 | 0 |
| 4 | 0 | 0 |
| 4 | 0 | 0 |
| $\overline{X} = 2.35$ | $\overline{X} = 0.10$ | $\overline{X} = 0.0$ |
| 5 animals | 5 animals | 5 animals |
| 10 horns | 10 horns | 10 horns |

Examples 4 and 5; TC-7, With LMW Heparin or HHS in Dried Form

In Examples 4 and 5, the procedures of Example 1 and 2 can be changed, so that the LMW heparin and HHS on TC-7 fabric are prepared in dried form. Two mls. of the solution were pipetted on a 3x4 inch piece of fabric (TC-7), which sat in an XT polymer tray, to completely saturate the fabric thus assuring uniform distribution. The wetted fabric was then allowed to sit overnight, during which time the solution flashed off leaving a dry, LMW heparin or HHS impregnated fabric.

The dried LMW heparin or HHS impregnated TC-7 fabric was then taken out of the tray and placed inside a Tyvek envelope (Grade 1013-B). This was in turn placed inside a foil laminate envelope (Maraflex -0.5 gauge Mylar, 0.00135 foil) which was itself sealed. The packages were then sterilized via use of 2.5 Mrads Cobalt irradiation.

Example 6 TC-7, With Heparinoid In Dried Form

The procedure of Example 1 is repeated except that heparinoid is substituted for LMW heparin. The dried heparinoid impregnated fabric produced is useful for adhesion prevention.

The above examples and data show that the addition of LMW heparin, heparinoid and HHS to an ORC fabric advantageously prevents surgical adhesions at the area of surgical incision in accordance with the methods of the invention.

The scope of the present invention is not limited by the description, examples and suggested uses herein and modifications can be made without departing from the spirit of the invention. Application of the compositions and methods of the present invention for medical and surgical uses can be accomplished by any suitable surgical and medical method and technique as is presently and prospectively know to those skilled in the art. Thus it is intended that the present invention cover the modifications and variations of this invention provided they come within the scope of the appended claims and their equivalents.

**Claims**

1. An improved adhesion-preventative barrier fabric comprising an oxidized regenerated cellulose fabric which is drapable, conformable, adherent to body organs, and substantially absorbable within thirty (30) days in the body, which fabric has a non-toxic, adhesion-preventative effective amount and potency of low molecular weight heparin, heparinoid, hexuronyl hexosaminoglycan sulfate or mixtures thereof absorbed thereon.

2. The product of claim 1 wherein the low molecular weight heparin, heparinoid or hexuronyl hexosaminoglycan sulfate absorbed on the fabric is applied to the fabric in liquid form at the time of actual use on a body organ during surgery.

3. The product of claim 1 wherein the low molecular weight heparin, heparinoid or hexuronyl hexosaminoglycan sulfate is absorbed on said fabric and subsequently dried.

4. The product of any preceding claim wherein the amount of low molecular weight heparin, heparinoid or hexuronyl hexosaminoglycan sulfate utilized is sufficient to saturate the fabric.

5. The product of any preceding claim wherein low molecular weight heparin is absorbed on the fabric.

6. The product of any of claims 1 to 4 wherein hexuronyl hexosaminoglycan sulfate is absorbed on the fabric.

7. The product of any of claims 1 to 4 wherein heparinoid is absorbed on the fabric.

8. The product of any preceding claim wherein the oxidized regenerated cellulose fabric is a knit fabric.

9. The product of any preceding claim wherein the low molecular weight heparin, heparinoid, or hexuronyl hexosaminoglycan sulfate is simply absorbed and not ionically bound to the barrier fabric.

10. The product of any preceding claim wherein the oxidized regenerated cellulose fabric is selected from the group consisting of hyaluronic acid, synthetic resorbable polymers, gelatin films, absorbable gel films, and oxidized cellulose fabrics.

11. An improved adhesion-preventative barrier material comprising a matrix which is safe for use in surgery and is drapable, conformable, adherent to body organs, and substantially absorbable within thirty (30) days in the body, which matrix has a non-toxic, adhesion-preventative effective amount and potency of lower molecular weight heparin, heparinoid, hexuronyl hexosaminoglycan sulfate or mixtures thereof absorbed thereon.

12. A barrier material according to any preceding claim for use in surgery.

13. The use of low molecular weight heparin, heparinoid, hexuronyl hexosaminoglycan sulfate or mixtures thereof in the manufacture of a barrier material for use in a method of surgery.

14. The use of low molecular weight heparin, heparinoid, hexuronyl hexosaminoglycan sulfate or mixtures thereof as claimed in claim 13, wherein said method comprises positioning said barrier material between the site of the surgical activity and neighbouring tissue.

15. The use of low molecular weight heparin, heparinoid, hexuronyl hexosaminoglycan sulfate or mixtures thereof as claimed in claim 14 wherein said barrier material comprises an oxidized, regenerated cellulose fabric which is drapable, conformable, adherent to body organs, and substantially absorbable within thirty (30) days in the body, which fabric has said low molecular weight heparin, heparinoid, hexuronyl hexosaminoglycan sulfate or mixtures thereof absorbed thereon.

16. The use of low molecular weight heparin, heparinoid, hexuronyl hexosaminoglycan sulfate or mixtures thereof as claimed in claim 15 wherein said low molecular weight heparin, heparinoid, hexuronyl hexosaminoglycan sulfate or mixture thereof is absorbed on the oxidized regenerated cellulose fabric after the fabric has been applied to said body organ.

17. The use of low molecular weight heparin, heparinoid, hexuronyl hexosaminoglycan sulfate or mixtures thereof as claimed in claim 15, wherein said low molecular weight heparin, heparinoid, hexuronyl hexosaminoglycan sulfate or mixture thereof is absorbed on the oxidized regenerated cellulose fabric before the fabric is applied to the body organ.

European Patent Office

EUROPEAN SEARCH REPORT

Application Number

EP 89 31 2780

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 262 890 (JOHNSON & JOHNSON) <br> * Page 3, lines 13-19; claims 1-13 * <br> ----- | 1-17 | A 61 L 31/00 |

TECHNICAL FIELDS SEARCHED (Int. Cl.5)

A 61 L

The present search report has been drawn up for all claims

| Place of search | Date of completion of the search | Examiner |
|---|---|---|
| THE HAGUE | 11-01-1990 | PELTRE CHR. |